## Europäisches Patentamt
## European Patent Office
## Office européen des brevets

(11) Publication number: **0 022 220**
**B1**

## EUROPEAN PATENT SPECIFICATION

(45) Date of publication of patent specification: **07.03.84**

(51) Int. Cl.³: **A 61 B 1/00, G 02 B 23/04**

(21) Application number: **80103615.3**

(22) Date of filing: **26.06.80**

(54) **Multiple viewing attachment for an endoscope.**

(30) Priority: **05.07.79 JP 85305/79**
**05.07.79 JP 85306/79**
**05.07.79 JP 85307/79**
**05.07.79 JP 85308/79**

(43) Date of publication of application:
**14.01.81 Bulletin 81/2**

(45) Publication of the grant of the patent:
**07.03.84 Bulletin 84/10**

(84) Designated Contracting States:
**BE CH DE FR GB IT LI NL SE**

(56) References cited:
**FR - A - 1 466 884**
**US - A - 3 729 252**
**US - A - 3 756 695**

(73) Proprietor: **OLYMPUS OPTICAL CO., LTD.**
**43-2, 2-chome, Hatagaya Shibuya-ku**
**Tokyo 151 (JP)**

(72) Inventor: **Terada, Masaaki**
**1667-130, Takane-machi Hachioji-shi**
**Tokyo (JP)**
Inventor: **Yamasita, Nobuo**
**20-5, Maruyama-machi Hachioji-shi**
**Tokyo (JP)**
Inventor: **Yamamoto, Kimiaki**
**7-14-11-132, Owada-machi Hachioji-shi**
**Tokyo (JP)**

(74) Representative: **Freischem, Werner, Dipl.-Ing. et al,**
**Patentanwälte Dipl.-Ing. W. Freischem Dipl.-Ing.**
**I. Freischem An Gross St. Martin 2**
**D-5000 Köln 1 (DE)**

Courier Press, Leamington Spa, England.

# Multiple viewing attachment for an endoscope

The present invention relates to a multiple viewing attachment for an endoscope.

An endoscope is provided with a multiple viewing attachment in order that a plurality of observers can observe an endoscope viewing image in diagnosis by the endoscope. In the structure, the multiple viewing attachment includes a main eyepiece and a subsidiary eyepiece which are coupled by means of a subsidiary optical guide. The light beam including an image, after passing through an image guide of the endoscope, is splitted into two optical paths by a half-mirror provided on the main eyepiece. Part of the image is converged at the incident terminal of the subsidiary optical guide. An image appearing at the light incident end of the subsidiary image guide is transmitted through the subsidiary image guide to the light output end and then is observed by the subsidiary eyepiece. At this time, same image may be observed by the main eyepiece. In this case, imaged on the light incident end of the subsidiary image guide are an image of the light output end of the image guide of the endoscope, that is, an arrangement pattern of the optical fibers, in addition to the above-mentioned endoscope viewing image. At this time, unless the arrangement pattern is exactly coincident with that of the optical fibers of the subsidiary image guide, a moire fringe appears in an image as viewed from the subsidiary eyepiece, so that the image is vague with a pattern of a dark and white distribution.

There is a known approach to prevent the moire fringe from appearing, in which the fiber arrangement pattern of the end surface of the endoscope image guide is optically coupled with that of the subsidiary image guide end surface of the multiple viewing attachment with the directions of the arrangement patterns being displaced by a given angle. To completely prevent the moire fringe by the approach, both the directions of the arrangement patterns must be arranged with a good regularity. Further, the endoscope and the multiple viewing attachment must reliably be attached so as to dislocate from their correct positions. This leads to complexity of the structures of the endoscope and the attachment, increase of the steps of the manufacturing process, and increase of the manufacturing cost. For the reason that the endoscope and the multiple viewing attachment must be integrally fixed, respectively, the attachment must be rotated even with the rotation of the endoscope. This results in a remarkable reduction of the operability of the endoscope and the multiple viewing attachment. Even when the end surfaces of the image guide of the endoscope and of the subsidiary image guide of the attachment are reliably fixed so as to prevent the moire fringe, if the fiber arrangement of each optical guide on the end surfaces are irregular, e.g. the fibers are arranged in a bent or wavy stage, the moire fringe still appears. Use of only the optical guides with well regularly arranged fibers at the end surface, leads to a high cost to manufacture due to its yield.

Accordingly, the object of the invention as claimed in claim 1 is to provide a multiple viewing attachment for an endoscope with a simple construction which is free from the moire;

according to the invention, there is a multiple viewing attachment for an endoscope with a spatial filter to remove the optical fiber arrangement pattern on the end surface of the image guide of an endoscope;

the said spatial filter provides a phase difference of 1/2 wave length of a light or odd number of times the 1/2 wave length in accordance with a pattern of the optical fiber arrangement on the end surface of the image guide of the endoscope.

FR—A—1 466 884 discloses a multiple viewing attachment having two viewers. One viewer is connected to a TV camera or to a cine camera. A coupling is used and the focal point can be adjusted to the TV camera or cine camera.

This invention discloses the prevention of moire fringes caused by the interference of the arrangement pattern of optical fiber bundle of the endoscope and that of the multiple viewing attachment.

US—P—3 729 252 is the same as this invention in that it discloses the prevention of moire fringes. However, this reference does not disclose the spatial filter which is applicable to various endoscopes as does this invention. Namely, in this invention a spatial filter is constructed by forming large and small coating spots in a transparent substrate. When this spatial filter is used in the multiple viewing attachment, moire fringes can be prevented even when the attachment is attached to a variety of different endoscopes.

Other objects and features of the present invention will be apparent from the following description taken in connection with the accompanying drawings, in which:

Fig. 1 is a plan view of a cross sectional view of a multiple viewing attachment for an endoscope according to the present invention;

Fig. 2 is a plan view of a spatial filter used for the multiple viewing attachment shown in Fig. 1;

Fig. 3 is an arrangement pattern of optical fibers of the image guide of an endoscope;

Fig. 4 is a schematic diagram of a multiple viewing attachment provided on the subsidiary optical guide of a spatial filter, which is another embodiment according to the present invention;

Figs. 5 to 7 are plan views of different spatial filters used in the multiple viewing attachment according to the present invention;

Fig. 8 is a schematic diagram of a multiple viewing attachment using an image pickup device, which is still another embodiment of the invention.

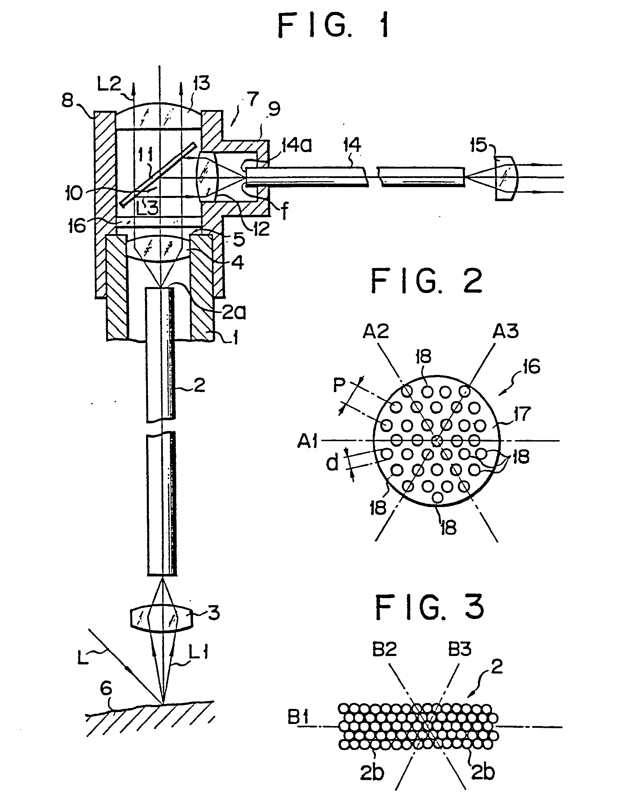

In Fig. 1 illustrating a multiple viewing attachment for an endoscope according to the invention, an endoscope 1 is provided with an image guide 2 and a light guide (not shown). The image guide 2 and the light guide are each a bundle of a number of optical fibers. The image guide 2 is provided on the side of one end surface with an objective lens 3 and faces on the side of the other end surface 2a an eyepiece lens 4. An eyepiece 5 with the eyepiece lens 4 is removably and rotatably provided with a main eyepiece 8 of the multiple viewing attachment 7. A subsidiary connecting portion 9 is formed on a part of the peripheral portion of the main eyepiece 8. An optical system 10 is provided in the main eyepiece 8. The optical system 10 is comprised of a beam splitter for splitting an optical image from the image guide 2 into a plurality of optical paths, for example, a half-mirror 11 disposed obliquely with respect to the optical axis of the image guide 2 of the endoscope 1, and a focusing lens 12 supported by the subsidiary connecting portion 9. An eyepiece 13 is provided on an optical path of the image guide 2. Mounted to the auxiliary connecting portion 9 is a subsidiary image guide 14. The subsidiary image guide 14 is a bundle of a number of optical fibers, like the image guide 2. The subsidiary image guide 14 leads an image formed at one end surface 14a of the image guide 14 to the subsidiary eyepiece lens 15.

A spatial filter 16 is disposed between the eyepiece lens 4 of the endoscope 1 and the half-mirror 11 within the multiple viewing attachment 7 and mounted to the main eyepiece 8. The spatial filter 16 as shown in Fig. 2 is comprised of a disc-like transparent substrate or plate 17 made of transparent plastic or transparent glass and transparent coatings 18 formed on the transparent plate 17 with much the same size as that of the substrate 17. The transparent coatings 18 are so constructed as to provide a phase difference by 1/2 wave length or by an odd number of the 1/2 wave length between the wave lengths of the light beams passing through the transparent plate 17 and the transparent coatings 18. The transparent coatings 18 are formed circularly by using magnesium fluoride ($MgF_2$) or cerium dioxide ($CeO_2$), for example. Those coatings 18 are arranged along three lines A1, A2 and A3 which are crossed at the center of the transparent plate 17 so as to divided it into six equal sectrial sections. An interval P between the adjacent coatings 18 is so selected as to be 1.5 times the effective diameter d of the coating 18. As the interval P is longer than the 1.5 d, it is better for preventing the moire fringe, but it attenuates the light beam. For this reason, approximately 1.5 d is preferable for the interval P. If it is less than 1.5 d, the moire fringe preventive effect is small.

The spatial filter 16 thus constructed has a frequency response in which the light intensity is remarkably suppressed in high frequencies and the light with frequencies higher than the cut-off frequency No is completely cut off. The light rays with the frequencies lower than the cut-off frequency No is capable of forming an image to be observed. On the other hand, the light rays with the frequencies higher than the cut-off frequency forms the moire fringe. Therefore, such light rays are removed by the spatial filter 16.

A pattern of the coatings formed on the spatial filter 16 is so formed that it has the following relation to a pattern of the arrangement of the optical fibers at the end surface 2a of the image guide 2. The arrangement pattern of the optical fibers appearing on the end surface of the image guide 2 has a pattern, as shown in Fig. 3, corresponding to the coating pattern of the spatial filter 16. Thus, lines B1, B2 and B3 along the arrangement pattern of the optical fibers, respectively, are coincident with the lines A1, A2 and A3.

In observing an image of the endoscope by two observers, for example, a doctor and an intern, by using the multiple viewing attachment 7 with such a construction, the doctor looks in by the main eyepiece 8 while the intern looks in by the subsidiary eyepiece 15. In this way, two observers can simultaneously observe the image. For the image formation, the light L transmitted through the light guide (not shown) illuminates an object 6, so that a reflecting light L1 from the object 6 is transmitted through an objective 3 to be imaged at the incident end of the image guide 2, as an image of the object. The image appears at the end surface 2a of the image guide 2, such that it superposes on the arrangement pattern of the optical fibers of the image guide 2. The composed image of the image of the object and the optical fiber arrangement pattern is imaged on the half mirror 11 by the eyepiece lens 4. In this case, the component of the arrangement pattern is removed from the composite image by the spatial filter 16. In other words, a dark portion of the arrangement pattern of the optical fiber, that is, a clad image at the portion between the adjacent optical fibers is removed by the spatial filter 16, so that the image formed on the half mirror 11 through the spatial filter does not include the arrangement pattern of the optical fiber 16. The image on the half mirror 11 is observed by a doctor through the eyepiece lens 13 while at the same time the same image is formed on the incident end surface of the subsidiary image guide 14 by means of the lens 14 and is observed by an intern through a subsidiary eyepiece lens 15. As described above, the fiber arrangement pattern of the image guide is removed by the spatial filter 16. As a result, no moire fringe is included in the image observed by the intern. Thus, the image obtained has an extreme high quality with no light and shade distribution of the moire fringe.

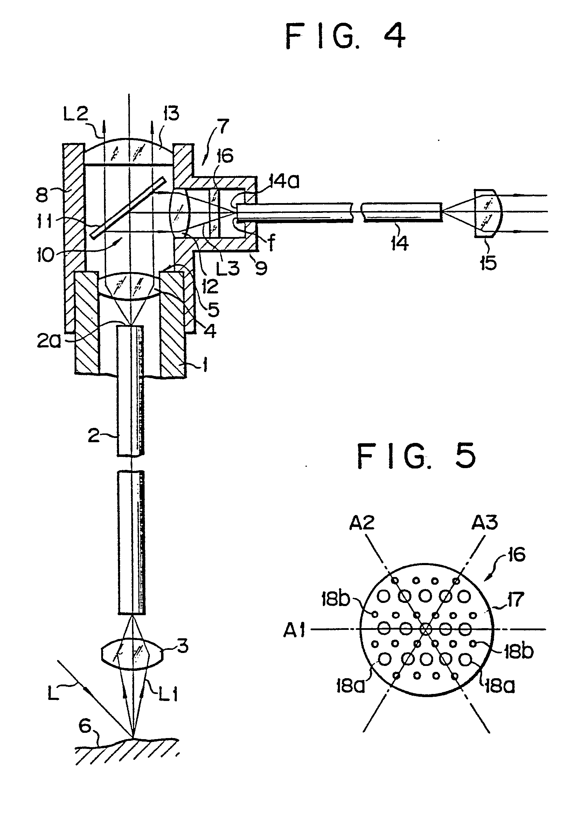

In the above-mentioned embodiment, the spatial filter 16 is provided between the eyepiece lens 14 of the endoscope 1 and the main eyepiece 8 of the multiple viewing attachment. Alternatively, the spatial filter 16 may be provided between the lens 12 provided at the subsidiary connecting portion 9 and the end surface of the subsidiary image guide 14, as shown in Fig. 4. The pattern of the coating formed on the spatial filter 16 is not limited to the pattern as shown in Fig. 2, but may be formed as shown in Figs. 5 to 7.

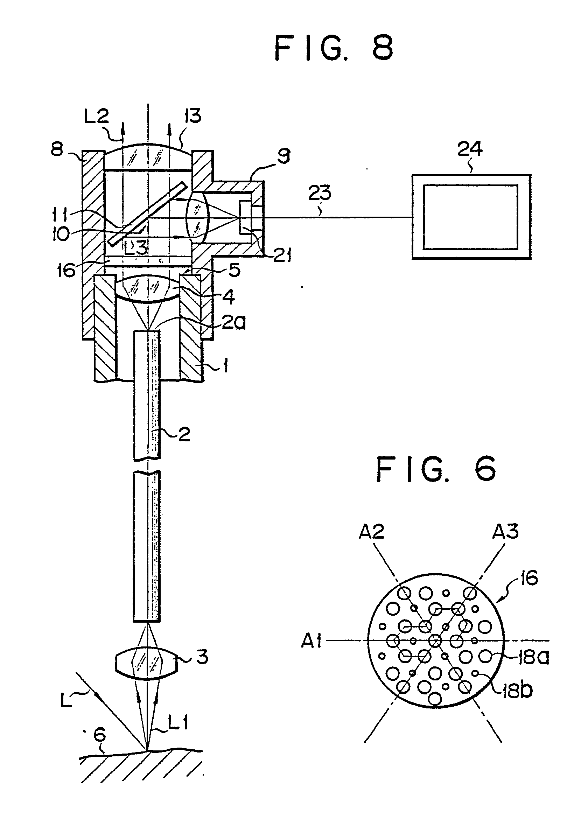

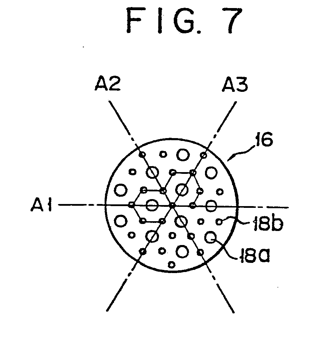

As seen from Fig. 5, transparent coatings 18a..., and 18b... with different sizes are formed on the transparent plate 17. The coatings 18a with large diameters are arranged along the line Al. Along lines A2 and A3, the large diameter coatings 18a and the small diameter coating 18b are alternately arranged, as shown. The sizes of the coatings 18a and 18b are optimally determined depending on the diameter of each optical fiber of the image guide of the endoscope 1. Generally, the diameter of the optical fiber varies in accordance with the kind of the endoscope 1 used. Therefore, if the coatings with different diameters are used, it is applicable for the endoscope of many different kinds. In a spatial filter shown in Fig. 6, the coatings 18a with large diameter are disposed hexagonally, surrounding the coatings 18b with small diameter. To the contrary, in a spatial filter shown in Fig. 7, the coatings 18b with small diameter are hexagonally disposed surrounding the large diameter coatings 18a. The hexagonal arrangement of the coatings may be replaced by an arrangement in which the coatings with large and small diameters are disposed octagonally or rectangularly. Additionally, the coatings which are small circles in the above-mentioned embodiment may take any shape such as rectangular and triangle. Additionally the coatings may be replaced by throughholes. If the throughholes are used, a spatial filter is constructed so that the phase of the light beam passing through the throughhole is displaced from that of the light beam passing through the transparent plate by 1/2 wave length or odd number times of the 1/2 wave length.

Turning now to Fig. 8, there is shown another embodiment of the invention in which a solid-state image pick-up device is used. According to the embodiment, the solid-state image pick-up device, for example, CCD, is mounted to the subsidiary connecting portion 9. The output terminal of the CCD 21 is connected to a monitor television 24 by way of a line 23. In this embodiment, an image with no arrangement pattern of optical fibers of the image guide 2 due to the action of the spatial filter 16 is formed on the pick-up surface of the CCD 21, so that an image picked up through the scanning operation of the CCD 21 has no moire fringe, with the result that an image with good quality is screened on the monitor television 24.

As described above, the spatial filter is provided on the optical path ranging from the light-emanating end surface of the image guide of the endoscope to the end surface of the subsidiary optical guide of the multiple viewing attachment or the incident surface of the CCD. The spatial filter has spot patterns for removing the arrangement pattern of the optical fibers of the image guide. The spatial filter provides the phase displacement of 1/2 wave length or an odd number of times of the 1/2 wave length between the light beams passing through the spot patterns and that passing through the remaining portion of the filter, thereby to remove the light rays with the wave length corresponding to the unnecessary frequency. With the provision of the spatial filter, the image to be observed by the eyepiece of the multiple viewing attachment is a good quality image with no moire fringe. Additionally, the multiple viewing attachment may rotatably be mounted to the eyepiece of the endoscope. This feature eliminates the need for moving the subsidiary eyepiece simultaneously with the operation. In this respect, the operability of the endoscope is enhanced. Furthermore, even if the arrangement of the optical fibers or the picture element arrangement of the CCD is marred, the occurrance of the moire fringe may be prevented. In manufacturing the endoscope and the multiple viewing attachment, there is no need for accurate alignment in the arrangement of the optical fibers and the picture elements of the CCD. This makes easy the manufacturing work of the device. The allowance of the high accuracy in the machining of parts reduces the cost of the device.

## Claims

1. A multiple viewing attachment removably attached to an endoscope (1) having at least an eyepiece and an image guide (2) formed of a number of optical fibers (2b) closely arranged with a given arrangement pattern for guiding an optical image to the eyepiece (5), said multiple viewing attachment comprising: a main eyepiece (8); at least one optical image receiving means (14, 15 or 21, 23, 24); a beam splitter (11) for splitting said optical image led by said image guide (2) of said endoscope (1) into at least two optical paths extending to said main eyepiece (8) and to said optical image receiving means, respectively; and optical means (16, 116) which is disposed on the optical paths to said optical image receiving means and removes an image corresponding to the arrangement pattern of said image guide of said endoscope from said optical image, said optical means is a spatial filter including transparent substrate (17) and a number of small and large transparent coating spots (18) formed on said transparent substrate correspondingly to the regularity of the arrangement pattern of said image guide of said endoscope, and having an optical characteristic to provide a phase difference between the light rays passing through the coating spots and those passing through said transparent substrate by 1/2 wave length or odd number times of the 1/2 wave length.

2. A multiple viewing attachment as set forth in claim 1, wherein said large diameter coating spots (18a) of said spatial filter are polygonally arranged surrounding each of said small diameter coating spots (18b).

3. A multiple viewing attachment as set forth in claim 1, wherein said small diameter coating spots (18b) are polygonally arranged surrounding each of said large diameter coating spots (18a).

4. A multiple viewing attachment as set forth in claim 2, wherein said spatial filter includes a plurality of rows of the large diameter coating spots (18a) and a plurality of rows of the small diameter coating spots (18b) each of which is disposed between within the successive rows of said large diameter coating spots (18a).

5. A multiple viewing attachment as set forth in any one of claims 1 to 4, wherein said coating spots (18, 18a, 18b) are made of material selected from a group magnesium fluoride and cerium dioxide.

6. A multiple viewing attachment as set forth in any one of claims 1 to 5, wherein said optical means is disposed between said eyepiece (15) of said endoscope and said beam splitter (11).

7. A multiple viewing attachment as set forth in claims 1 to 6, wherein said optical means is disposed between said beam splitter (11) and said optical image receiving means (14, 15 or 21).

8. A multiple viewing attachment as set forth in any one of claims 1 to 7, wherein said optical image receiving means includes a subsidiary eyepiece (15), a subsidiary image guide (14) optically coupled with said subsidiary eyepiece with said main eyepiece (8).

9. A multiple viewing attachment as set forth in any one of claims 1 to 8, wherein said optical image receiving means comprises a solid-state image pick-up device.

**Revendications**

1. Accessoire de visée multiple fixé de manière amovible sur un endoscope (1) comportant au moins un oculaire et un guide (2) d'image formé d'un certain nombre de fibres optiques (2b) étroitement disposées selon un modèle de disposition déterminé pour guider une image optique jusqu'à l'oculaire (5), cet accessoire de visée multiple étant caractérisé en ce qu'il comprend un oculaire principel (8); au moins un moyen (14, 15 our 21, 23, 24) de réception de l'image optique; un diviseur (11) de faisceau destiné à diviser l'image optique amenée par le guide (2) d'image de l'endoscope (1) en au moins deux trajets optiques se prolongeant respectivement jusqu'à l'oculaire principal (8) et jusqu'au moyen de réception de l'image optique; et un moyen optique (16, 116) qui est monté sur les trajets optiques vers le moyen de réception de l'image optique, et qui élimine de l'image optique une image correspondant au modèle de disposition du guide d'image de l'endoscope, ce moyen optique étant un filtre spatial comprenant un substrat transparent (17) et un certain nombre de zones (18) de dépôts transparents de petit et de grand diamètres formées sur ce substrat transparent de manière à correspondre à la régularité du modèle de disposition du guide d'image de l'endoscope, et ayant une caractéristique optique qui produit un déphasage d'une demi-longueur d'onde ou d'un multiple impair de la demi-longueur d'onde entre les rayons lumineux traversant les zones de dépôt et ceux qui traversent le substrat transparent.

2. Accessoire de visée multiple suivant la revendication 1, caractérisé en ce que les zones (18a) de dépôts de grand diamètre du filtre spatial sont disposées en forme de polygone autour de chacune des zones (18b) de dépôts de petit diamètre.

3. Accessoire de visée multiple suivant la revendication 1, caractérisé en ce que des zones (18b) de dépôts de petit diamètre sont disposées en forme de polygone autour de chacune des zones (18a) de dépôts de grand diamètre.

4. Accessoire de visée multiple suivant la revendication 2, caractérisé en ce que le filtre spatial comprend une pluralité de rangées de zonnes (18a) de dépôts de grand diamètre et une pluralité de rangées de zones (18b) de dépôts de petit diamètre, disposées chacune entre les rangées successives de zones (18a) de dépôts de grand diamètre.

5. Accessoire de visée multiple suivant l'une quelconque des revendications 1 à 4, caractérisé en ce que les zones (18, 18a et 18b) de dépôts sont constituées d'une matière sélectionnée parmi un groupe formé du fluorure de magnésium et du bioxyde de cérium.

6. Accessoire de visée multiple suivant l'une quelconque des revendications 1 à 5, caractérisé en ce que le moyen optique est monté entre l'oculaire (15) de l'endoscope et le diviseur (11) de faisceaux.

7. Accessoire de visée multiple suivant les revendications 1 à 6, caractérisé en ce que le moyen optique est monté entre le diviseur (11) de faisceaux et le moyen (14, 15 ou 21) de réception de l'image optique.

8. Accessoire de visée multiple suivant l'une quelconque des revendications 1 à 7, caractérisé en ce que le moyen de réception de l'image optique comprend un oculaire auxiliaire (15), un guide auxiliaire (14) d'image optique- ment couplé à l'oculaire auxiliaire et à l'oculaire principal (8).

9. Accessoire de visée multiple suivant l'une quelconque des revendications 1 à 8, caractérisé en ce que le moyen de réception de l'image optique comprend un dispositif de captage de l'image entièrement transistorisé.

**Patentansprüche**

1. Mehrfachokularansatz, der lösbar an einem Endoskop (1) angebracht ist, welches wenigstens ein Okular und einen Bildleiter (2) aufweist, welcher, um ein optisches Bild zum Okular (5) hinzuleiten, aus einer Anzahl von in einem bestimmten Anordnungsmuster dicht gepackten optischen Fasern (2b) gebildet ist, gekennzeichnet durch ein Hauptokular (8); wenigstens eine optische Bildaufnahmevorrichtung (14, 15 oder 21, 23, 24); einen Lichtstrahlteiler (11), der das durch den Bildleiter (2) des Endoskops (1) geleitete optische Bild in wenigstens zwei zum Hauptokular (8) bzw. zur optischen Bildaufnahmevorrichtung hin verlaufende optische Wege aufteilt; eine in den optischen Wegen zur optischen Bildaufnahmevorrichtung hin liegende optische Einrichtung (16, 116), welche aus dem optischen Bild ein dem Anordnungsmuster des Bildleiters des Endoskops entsprechendes Bild entfernt, wobei diese optische Einrichtung ein Phasenfilter mit einem durchsichtigen Substrat (17) und einer Anzahl kleiner und großer durchsichtiger Punktauflagen (18) ist, die auf dem durchsichtigen Substrat der Regelmäßigkeit des Anordnungsmusters des Bildleiters des Endoskops entsprechend vorgesehen sind und eine optische Charakteristik haben, welche zwischen den durch die Punktauflagen hindurchtretenden un den durch das durchsichtige Substrat hindurchtretenden Lichtstrahlen einen Phasenunterschied von einer 1/2 Wellenlänge oder einem ungeradzahligen Vielfachen der 1/2 Wellenlänge erzeugt.

2. Mehrfachokularansatz nach Anspruch 1, dadurch gekennzeichnet, daß die einen großen Durchmesser aufweisenden Punktauflagen (18a) des Phasenfilters um jede einen kleinen Durchmesser aufweisende Punktauflage (18b) herum polygonal angeordnet sind.

3. Mehrfachokularansatz nach Anspruch 1, dadurch gekennzeichnet, daß die einen kleinen Durchmesser aufweisenden Punktauflagen (18b) um jede einen großen Durchmesser aufweisende Punktauflage (18a) herum polygonal angeordnet sind.

4. Mehrfachokularansatz nach Anspruch 2, dadurch gekennzeichnet, daß das Phasenfilter mehrere Reihen von Punktauflagen (18a) großen Durchmessers aufweist sowie mehrere Reihen von Punktauflagen (18b) kleinen Durchmessers, deren jede zwischen zwei aufeinanderfolgenden Reihen von Punktauflagen (18a) großen Durchmessers gelegen ist.

5. Mehrfachokularansatz nach einem der Ansprüche 1 bis 4, dadurch gekennzeichnet, daß dei Punktauflagen (18, 18a, 18b) aus Magnesiumfluorid und/oder Cerdioxid hergestellt sind.

6. Mehrfachokularansatz nach einem der Ansprüche 1 bis 5, dadurch gekennzeichnet, daß die optische Einrichtung zwischen dem Okular (15) des Endoskops und dem Lichtstrahlteiler (11) angeordnet ist.

7. Mehrfachokularansatz nach einem der Ansprüche 1 bis 6, dadurch gekennzeichnet, daß die optische Einrichtung zwischen dem Lichtstrahlteiler (11) und der optischen Bildaufnahmevorrichtung (14, 15 oder 21) angeordnet ist.

8. Mehrfachokularansatz nach einem der Ansprüche 1 bis 7, dadurch gekennzeichnet, daß dei optische Bildaufnahmevorrichtung ein Zweitokular (15) und einen zweiten Bildleiter (14) aufweist, der mit dem Zweitokular und dem Hauptokular (8) optisch gekoppelt ist.

9. Mehrfachokularansatz nach einem der Ansprüche 1 bis 8, dadurch gekennzeichnet, daß die optische Bildaufnahmervorrichtung eine festkörperphysikalische Bildfängervorrichtung aufweist.

# FIG. 1

# FIG. 2

# FIG. 3

# F I G. 4

# F I G. 5

# FIG. 8

# FIG. 6

# FIG. 7